# EUROPEAN PATENT SPECIFICATION

(11) **EP 1 449 828 B1**
(45) Date of publication and mention of the grant of the patent: **18.04.2007**
(21) Application number: 04003617.0
(22) Date of filing: 30.08.1995
(51) Int. Cl.: C07C 279/14, A61K 31/215, A61K 31/275

(54) **Guanidinomethyl cyclohexane carboxylic acid ester derivatives**
Guanidinomethylcyclohexancarbonsäure-Ester Derivate
Dérivés d'esters de l'acide guanidinométhylcyclohexane

(30) Priority: 30.08.1994 JP 24348994; 30.08.1994 JP 24349094; 05.09.1994 JP 24827094; 09.09.1994 JP 25265594
(43) Date of publication of application: 25.08.2004
(62) Divisional of application: 99124756.0
(73) Proprietor: Nagase ChemteX Corporation, Osaka 550-8668 (JP)
(72) Inventor: Kamoda, Osamu, Itami-shi, Hyogo 664 (JP); Fujiwara, Hiromichi, Itami-shi, Hyogo 664 (JP); Yanagi, Toshiharu, Itami-shi, Hyogo 664 (JP)
(74) Representative: Schnappauf, Georg

(56) References cited:
- EP-A- 0 585 130
- FR-A- 2 498 183
- GB-A- 2 058 773

## Description

### 1. Field of the Invention

The present invention is related to novel compounds comprising an antibacterial action, especially with a strong antibacterial action against helicobacter pylori, and pharmaceutically acceptable salts thereof.

### 2. Background

Helicobacter pylori has been a remarked bacterium for investigating lesions of the stomach and duodenum for the report by Wallen and Marshal, in Australia, in 1983 (Lancet ii: 1437-1442 (1983)). It has been known that Helicobacter pylori is a helicoid microaerophile gram-negative bacillus, adjusts its life environment by producing urease, and lives in the tunica mucosa of the stomach and duodenum, and also causes or recauses inflammations or ulcerations.

It was reported that the United States Public Health Research Institute presented the recommendation to use a combination of antiulcerative agents and antibacterial agents for extermination of Helicobacter pylori (Chem. Indust. Daily Rep. Heisei 6.7.18) because Helicobacter pylori is considered to be strongly correlated with crisis and recurrence of peptic ulcer.

Antibiotics such as amoxicillin, cephalexin, clarislomycin (Jap. Clinic, vol. 51, No. 12, 1993), and synthetic antibacterial agents, such as ophroxacine, cyprofloxane, (APMIS, Suppl., 1007-1014, 1992), have been known as usable compounds for extermination of Helicobacter pylori and bacteria expulsion. Minocyline has also been known to be used for diseases caused by Helicobacter pylori, such as gastritis (Japanese Patent Publication No. Hei 6-508635 (95/508635)).

In Japan too, while the relation between Helicobacter pylori and peptic ulcer has been studied from now on, a movement has been raised, which investigates the response of Helicobacter to the combination of antibiotics such as amoxicillin and omeplazol, lansoplazol which inhibit the activity of a proton pump and are used in clinics as antiulceration agents.

Since Helicobacter pylori is a bacterium producing urease and adjusts its life environment with the urease activity, a method for solving the above problem by using compounds with an inhibitory action against the above urease activity has been known. For example, Japanese Patent Laid-Open No. 4-217950/1992 reports that the aminomethylcyclohexane carboxylic acid phenyl ester group comprises the inhibitory activity against the urease activity, and also an inhibitory action of proteases, and therefore it discloses compounds useful as drugs which are antiulceration agents. In the above publication, a compound of which an amino group was changed to a guanidino group, or a compound shown in the following Formula (XIV) was disclosed, and it was reported that the above mentioned compounds showed 88.7% of ulceration restraining ratio against an ethanol hydrochloride ulceration.

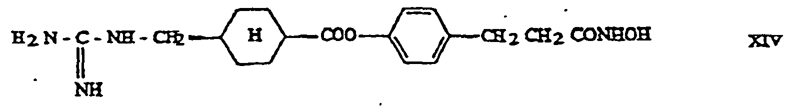

However, the above publication does not clarify the urease inhibitory activity of the compounds.

Further, in Japanese Patent Laid-Open No. 7-118153/1995, 2-[4[(3-methoxypropoxy)-3-methylpyridine-2-yl]methylsulfinyl-1H-benzimidazol, shown in Formula (XV) was disclosed as a compound with the inhibitory activity against urease.

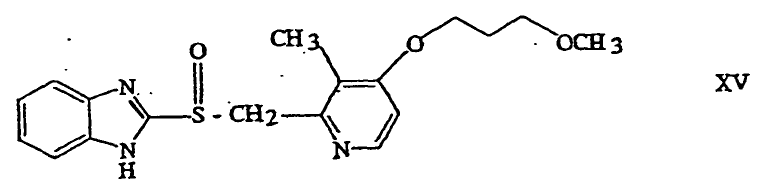

As administered antibiotics and synthetic antibacterial agents pass through the digestive tract, are absorbed in intestine, are metabolically distributed among going into blood, are excreted with feces and so on, the above drugs when passing through the intestine cause extinction of many bacteria living in the intestine and disturb the balance of enterobacteria. Therefore, the longer-term dosage of such drugs should be avoided. Accordingly, compounds which comprise selectively strong antibacterial agents against Helicobacter pylori have been desired.

As Helicobacter pylori is a bacterium living in the stomach, antibacterial activity of compounds having an antibacterial activity component which acts effectively in the stomach, becomes weak as the compounds move from the duodenum to the small intestine and, after all, it would be expected that the antibacterial activity vanishes. A compound called benexarte hydrochloride has been known as a compound which is chemically stable in the stomach, and is decomposed as moving to the small intestine (Progress in Medicine vol. 6, extra edition No. 1, 442 (1986)). It is reported that the anti-Helicobacter pylori activity of this compound was MIC 25-50 µg/ml (Disease of systema digestorium and Helicobacter pylori, P 91, Medical Review).

The present inventors took into consideration the above mentioned background and studied compounds with specific and effective antibacterial activity against Helicobacter pylori and have achieved the present invention. The present invention provides novel compounds comprising superior growth inhibition ability against Helicobacter pylori, but do not have an activity against Esylhiacori, Staphylococcus aureus, methacycline resistant bacteria, and so on, and are extremely speedy decomposed by actions of decomposition enzymes in the intestinum or blood.

U.S. Patent No. 4,220,662 discloses guanidino methyl cyclohexane carboxylic acid. As its ester, U.S. Patent No. 4,348,410 discloses a phenylester substituted with a group selected from halogen, lower alkoxy, formyl, lower alkanoylphenyl or a group of the formula -(CH₂)ₙCOOR3 which was useful as an anti-ulceration agent and as a protease inhibitor, wherein R3 of said formula -(CH₂)ₙCOOR3 is hydrogen, lower alkyl, phenyl, anisyle or alkoxycarbonylmethyl, and n is from 0 to 2. U.S. Patent No. 4,478,995 discloses that 6-benzyloxy carbonylphenyl ester is a useful antiulceration agent. Furthermore, it has also been well-known that such esters are useful as proteolytic enzyme inhibitors or antiulceration agents (Japanese Patent Publication No. 63-24988/1988, Japanese Patent Publication No. 63-24994/1988, Japanese Patent Publication No. 63-1940/1988, Japanese Patent Publication No. 63-32065/1988, Japanese Patent Publication No. 63-2255/1988, Japanese Patent Laid-Open No. 57-48960/1982, Japanese Patent Publication No. 64-2102/1989, Japanese Patent Publication No. 63-46743/1988, Japanese Patent Publication No. 64-2103/1989, Japanese Patent Publication No. 2-4588/1990, Japanese Patent Publication No. 64-2089/1989, Japanese Patent Publication No. 64-2086/1989, Japanese Patent Publication No. 64-2084/1989).

Moreover, it has also been well-known that a phenyl ester which is substituted with a halogen, lower alkyl, cyano, phenyl, benzyloxycarbonyl or phenoxycarbonyl group is useful against Escherichia coli (M. Kato et al., Biol. Pharma. Bull., 16 (2), 120-124 (1993)).

### 3. Detailed description of the Invention

The present invention relates to a compound shown in the Formula (III) 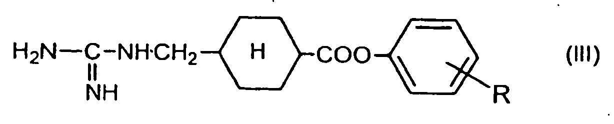 wherein R is selected from the group consisting of cycloalkyl group having three to eighteen carbon atoms, aralkyl group having seven to eighteen carbon atoms, arylalkenyl group having eight to eighteen carbon atoms, and substituted phenoxy group,
or pharmaceutically acceptable salts thereof.

Furthermore, the present invention relates to a compound shown in the Formula (IV) 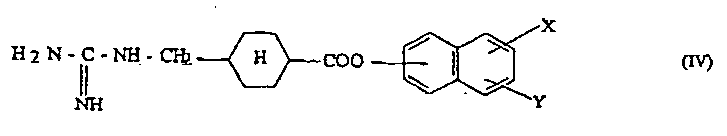 wherein X and Y are selected from the group consisting of hydrogen and alkoxycarbonyl group having two to nineteen carbon atoms respectively, with the proviso that X and Y are not both hydrogen,
or pharmaceutically acceptable salts thereof.

In addition, the present invention relates to a compound selected from the group consisting of trans-4-guanidinomethyl cyclohexane carboxylic acid (6-ethoxycarbonyl-2-naphthylester), trans-4-guanidinomethyl cyclohexane carboxylic acid (2,4-dichloro-1-naphthylester), trans-4-guanidinomethyl cyclohexane carboxylic acid (6-bromo-2-naphthylester), trans-4-guanidinomethyl cyclohexane carboxylic acid (4-cyclohexylphenylester), and trans-4-guanidinomethyl cyclohexane carboxylic acid (4-styrylphenylester), and pharmaceutically acceptable salts thereof.

Halogen according to the invention includes chlorine, iodine, bromine and fluorine.

Preferably, an alkyl group having one to eighteen carbon atoms is an alkyl group having from one to ten carbon atoms, more preferably an alkyl group having one to five carbon atoms, and may be linear or branched. Examples include methyl group, ethyl group, n-propyl group, i-propyl group; n-butyl group, i-butyl group, t-butyl group and so on.

Preferably, an alkoxy group having one to eighteen carbon atoms is an alkoxy group having one to ten carbon atoms, more preferably an alkoxy group having one to five carbon atoms, and may be linear or branched. Examples include methoxy group, ethoxy group, n-propoxy group and so on.

Preferably, a cycloalkyl group having three to eighteen carbon atoms is a cycloalkyl group having three to ten carbon atoms. Examples include cyclopropyl group, cyclobutyl group, cyclohexyl group and so on.

Preferably, an aralkyl group having seven to eighteen carbon atoms is an aralkyl group having seven to ten carbon atoms, and the alkyl group may be linear or branched. Examples include benzyl group, phenethyl group and so on.

Preferably, an arylalkenyl group having eight to eighteen carbon atoms is an arylalkenyl group having eight to ten carbon atoms, and may be linear or branched. Examples include styryl group and so on.

Substituents of the substituted phenoxy group are, e.g., halogens, cyano group, nitro group, carboxyl group, alkyl group having one to ten carbon atoms, preferably one to five carbon atoms, alkoxy group having one to ten carbon atoms, preferably one to five carbon atoms, trihalogenomethyl or alkoxycarbonyl group having two to nineteen carbon atoms, preferably two to eleven carbon atoms is suitable. Examples include fluorophenoxy group, 4-carboxyphenoxy group, 4-methoxycarbonylphenoxy group, 4-ethoxycarbonylphenoxy group.

Preferably, an alkoxy carbonyl group having two to nineteen carbon atoms is an alkoxy carbonyl group having two to eleven carbon atoms and may be linear or branched. Examples include methoxycarbonyl, ethoxycarbonyl, n-propyloxycarbonyl and i-propyloxycarbonyl.

As compounds of the present invention shown as the above mentioned Formula (III) and (IV) have nitrogen atoms respectively, the compounds of the present invention can form addition salts with various acids. These addition salts with various acids can be any salts which are pharmaceutically acceptable salts. Examples are hydrochloride, bromate, carbonate, acetate, methansulfonate, ethansulfonate, benzensulfonate, toluenesulfonate, oxalate, fumarate, coumarate, and so on. These various acid salts of the present invention can be exchanged with each other, and, in this case the step via a carbonate salt is suitable.

Compounds of the present invention or the pharmaceutically acceptable salts thereof can be produced by the reaction between guanidinomethyl cyclohexane carboxylic acid shown as Formula (V) or the reactive derivatives thereof and the compounds shown as Formula (IX) or (X) in a suitable solvent. 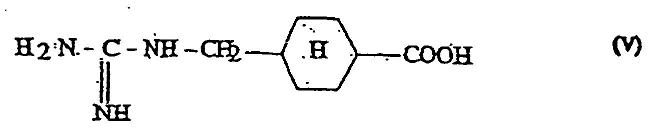 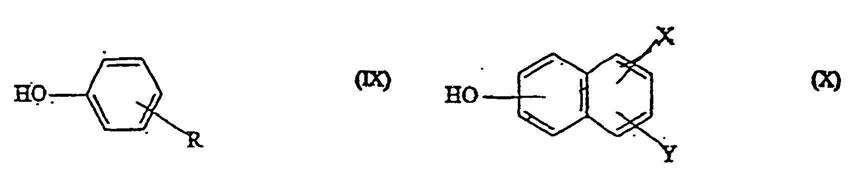 wherein R, X and Y are defined as above.

The suitable solvent for the above reaction can be any solvent which does not react with the starting materials. As such solvents, tetrahydrofuran, dioxane, isopropyl ether, ethylene glycol dimethyl ether, benzene, toluene, xylene, hexane, heptane, octane, petroleum ether, dichloroethane, acetonitrile, dimethylformamide, pyridine, triethylamine, dimethylaniline or mixture thereof and so on may be used.

As reactive derivative of the compound of Formula (V), for example, acid halide (chloride, bromide etc.), active ester (with p-nitrophenol etc.), acid anhydride, mixed acid anhydride (with ethyl chlorocarbonate, acetyl chloride, pivalic acid, POCl₃ etc.), and the reaction product with 1,1'-sulfinidimidazole, 1,1'-carbodiimidazole and so on may be used.

Condensation agents may be used to react the compound of Formula (V) having the free carboxyl group with the compound of Formula (IX) or (X). As suitable condensation agents, for example, dicyclohexylcarbodiimide, sulfuric acid, hydrogen chloride, toluensulfonic acid, thionyl chloride, phosphorus chloride, boron trifluoride and so on may be used.

Further, a reactive derivative of the compound of Formula (IX) or (X) may be used to react with the compound of Formula (V). Exemplary reactive derivatives of the compound of Formula (IX) or (X) are, e.g., chloro sulfate derivatives derived from thionylchloride, or sulfurous esters shown as Formula (VII).

**Ar-O-SO-O-Ar** **(VII)**

Compounds of Formula (V) used as starting materials also include acid addition salts. These acid addition salts are the same as the above mentioned pharmaceutically acceptable salts.

As the compounds of Formula (III) or (IV) include a cyclohexane ring, the guanidinomethyl group thereof and the carbonyl group thereof can be in trans configuration or cis configuration at ends of the cyclohexane ring. The compounds of the present invention and the pharmaceutically acceptable salts thereof also include these both types of compounds, preferred is the trans type. The configuration depends on that of the compound of Formula (V) used as a starting material.

The compounds of the present invention are useful in the treatment of patients infected with Helicobacter pylori.

The oral administration dose of the compounds of the present invention, which depends on the patients and symptoms, may be 10-200 mg/dose, and the compounds can be administered two or three times per day. Pharmaceutical forms thereof may be any form which can be orally administered. Examples include tablets, pellets, powder, granules, capsules, syrup or other solutions. For example, preparation can be prepared according to the pharmaceutical general rules in the Japanese Pharmacopoeia.

The excipients for obtaining solid preparations may be, for example, saccharides, such as saccharose, milk sugar, mannitol, glucose, starch and starch derivatives, crystal cellulose, low-substituted hydroxy propylcellulose, calcium hydrogenphosphate, calcium sulfate, calcium lactate, calcium citrate, aliphatic esters of sucrose, polyoxyethylene-polyoxypropyleneglycols and so on. Decay agents may be starch, calcium carbonate, carmelose and the salts thereof. Binding agents may be polyvinylalcohol, polyvinylpyrrolidone, hydroxyethylcellulose, hydroxypropylmethylcellulose, hydroxypropylcellulose, macrogols, gum arabic, gelatin, starch and so on. Glossing agents may be talc, magnesium stearate, calcium stearate, colloidal silica, stearic acid, waxes, hardened oil, polyethyleneglycols and so on.

The solution agent for preparing suspension agents, such as lyophobic agents, include, for example, gum arabic, gum xanthene, gelatin, sodium alaginic acid, Borbitol, glycerin, cane sugar or cellulose derivatives, such as sodium carmelose, methylcellulose and so on. Preservative agents may be benzoic acid, paraoxy benzoic acid esters and so on.

Edulcorant agents may be saccharose, sodiumsaccharine, sorbitol and so on. Flavor agents may be citric acid, peppermint oil, eucally oil and so on.

The antibacterial action of the compounds of the present invention are examined as follows.

### 1. Minimum Inhibitory Concentration (MIC) was measured in accordance with the standard method of Japanese Chemotherapy Academy (Chemotherapy, 29: 76-79, 1981).

### 1) Measuring Methods of Sensitivity

Sensitivity was measured with an agar plating dilution using Brain-Heart Infusion agar (Difco Co.) to which 0.1% of cyclodextrin was added.

### 2) Stepwise Concentrations of Antibacterial Agents

The prepared stepwise concentrations were 25, 12.5, 6.25, 3.13, 1.56, 0.78, 0.39, 0.20, 0.10, 0.05, 0.025 and 0.0125 µg/ml

10,000 µg/ml solutions of the compounds which were obtained in the examples described below were prepared by dissolving in dimethylsulfoxide. The solutions were diluted with sterilized distilled water to 250,125, 62.5, 31.3,15.6, 7.8, 3.9, 2.0,1.0,0.5,0.25 and 0.125 µg/ml. Each of 1 ml of these solutions was dispensed in share, after sterilization, 9 ml of sensitivity measuring medium which was kept at 50°C was added to each, and they were sufficiently mixed. The mixture was used as a plate for measuring sensitivity.

### 3) Bacterial Solution for Inoculation

Brain Heart Infusion agar medium (Difco Co.), to which 0.1% of cyclodextrin was added, was used as bacteria growth medium. The bacteria were cultured under microair at 37°C for three days, and the number of bacteria adjusted to about 106/ml. This was used as a bacterial solution for inoculation.

### 4) Method of Inoculating Bacteria

The medium was spread in each square about 2cm with a nichromic line loop.

### 5) Period and Temperature of the Culture

The medium was cultured under microair for three days at 37°C.

### 6) Evaluation

The minimum concentration which completely inhibited growth of the bacteria in the medium was judged as the value of MIC.

### 2. Minimum Inhibitory Concentration (MIC) and Minimum Bactericidal

Concentration (MBC) against Helicobacter pylori were measured in accordance with the liquid medium dilution.

1,000 µg/ml solutions of the compounds which were obtained in the examples described below were prepared by dissolving in dimethylsulfoxide (DMSO). Distilled water was added to the solutions to prepare a double dilution system (200, 100, ..., 0.05 µg/ml). 1 ml of Brain-Heart Infusion broth (Difco Co.) to which 0.1% of cyclodextrin was added, and then was prepared at double concentrations, was added to each of 1 ml of the above DMSO solutions and mixed. 10 µl of Helicobacter pylori bacterial solution, which was cultured at 37°C for three days and then diluted and prepared with the above mentioned liquid medium, was added to the mixtures to adjust the volume to 10⁵ CFU/mL The mixture was cultured in a multi-gas incubator (10% CO₂, 5% O₂ 85% N₂) at 37°C for three days and the minimum concentration of the compounds at which no growth of the bacteria was recognized with naked eyes were the values of MIC.

After the evaluation of MIC, each 100 µl of the compounds in each test tube were added to 4 ml of new liquid medium respectively. After culturing by the same method as described above, the minimum concentration of the compounds at which no growth was recognized with naked eyes were the values of MBC.

The present invention will be described more specifically with several examples.
The following examples do not limit the present invention. Those compounds which are not encompassed by the claims are given for comparative purposes only.

### 3. Best modes of operation of the Invention

As comparative samples, the minimum inhibitory concentration (MIC) of the following well-known compounds other than the compounds of the examples were measured by the same procedure as described above.
Compound 1: trans-4-guanidinomethyl cyclohexane carboxylic acid (4-phenylphenylester) hydrochloride
Compound 2: trans-4-guanidinomethyl cyclohexane carboxylic acid (2-phenylphenylester) hydrochloride
Compound 3: trans-4-guanidinomethyl cyclohexane carboxylic acid (1-naphthylester) hydrochloride
Compound 4: trans-4-guanidinomethyl cyclohexane carboxylic acid (2-naphthylester) hydrochloride

The values of MIC of Compounds 1 and 2 are shown in Table 1, the values of MIC of the compounds of Examples 1-3 and Compounds 3 and 4 are shown in Table 2, the values of MIC of the compounds of Examples 4-9 are shown in Table 3, and the value of MIC of the compound of Example 7 is shown in Table 4.

As a comparison, MICs of ophroxacine and amoxicillin were measured and the results are shown in Tables 1, 3, and 4. The antibacterial activity against Escherichia coli of the compounds of Examples 1-9 and Compounds 1-4 was also measured in accordance with the standard method of Japanese Chemotherapy Academy. The results are shown in the corresponding tables.

The antibacterial activity of Compound 1 was measured using medium with 7% horse's defiber blood, which is the product been on the market, instead of 0.1% cyclodextrin used as the sensivity of measuring medium for the measurement of antibacterial activity. As the results, the values of MIC were >25 µg/ml and >25 µg/ml respectively

For reference, the value of MIC of benexart hydrochloride was >100 µg/ml.

MIC of the compound of Example 7 was measured. The result is shown in Table 4.

### Example 1

Cooling down with ice, 0.41g of thionyl chloride was added by dropping to a mixture of 1.50g of 6-hydroxy-2-naphthoic acid ethylester, 0.55g of pyridine and 30ml of dried tetrahydrofuran. After the mixture was stirred for 4 hours at room temperature, precipitated crystals were collected by filtration. The filtrate was concentrated under reduced pressure and crystals were obtained. 30ml of dried pyridine was added to the crystals and the mixture was cooled down with ice. 0.74g of trans-4-guanidinomethyl cyclohexane carboxylic acid · hydrochloride was added to the cooled mixture in portions. After stirring this mixture overnight at room temperature, the mixture was concentrated under reduced pressure. Acetone was added to the residue, and precipitated crystals were collected by filtration, and washed with acetone. 1.03g of trans-4-guanidinomethyl cyclohexane carboxylic acid 6-ethoxycarbonyl-2-naphthylester) · hydrochloride was obtained.
mp 196-200°C; NMR (DMSO-d₆) δ 0.8-3.3 (12H,m), 1.40 (3H,t), 4.38 (2H,q), 7.0-8.8 (11H,m)

### Example 2

Trans-4-guanidinomethyl cyclohexane carboxylic acid · hydrochloride 2.00 g was suspended in 30ml of dried pyridine, and added with 1.99g of 2,4-dichloro-1-naphthol and 1.93 g of N,N'-dicyclohexylcarbodiimido. The mixture was stirred for 4 hours at 40°C. After cooling down with water, precipitated crystals were collected by filtration. The obtained crude crystals were suspended in chloroform three times repeatedly. 2.99g of trans-4-guanidinomethyl cyclohexane carboxylic acid (2,4-dichloro-1-naphthylester) · hydrochloride was obtained.
mp 208-209°C; NMR (DMSO-d₆) δ 0.6-3.3 (12H,m), 7.30 (4H,bs), 7.5-8.4 (6H,m)

### Example 3

Cooling down with ice, 1.1 1g of thionyl chloride was added by dropping to a mixture of 4.16g of 6-bromo-2-naphthol, 1.48g of dried pyridine and 30ml of dried tetrahydrofuran. After the mixture was stirred for 3 hours and cooled down with ice, precipitated crystals were collected by filtration. The filtrate was concentrated under reduced pressure. 30ml of dried pyridine was added to the residue and the mixture was homogenized and cooled down with ice. 2.00g of trans-4-guanidinomethyl cyclohexane carboxylic acid hydrochloride was added to the cooled mixture in portions. After stirring this mixture during night at room temperature, precipitated crystals were collected by filtration, and washed with acetone. 0.90g of trans-4-guanidinomethyl cyclohexane carboxylic acid (6-bromo-2-naphthyl ester) · hydrochloride was obtained.
mp 185-186.5°C; NMR (DMSO-d₆) δ 0.5-3.3 (12H,m). 6.7-8.5 (11H,m)

### Example 4

Cooling down with ice, 1.67g of thionyl chloride was added by dropping to a mixture of 4.94g of 4-cyclohexyphenol, 2.21g of pyridine and 30ml of dried tetrahydrofuran. After the mixture was stirred for 4 hours at room temperature, precipitated crystals were collected by filtration. The filtrate was concentrated under reduced pressure and an oily product was obtained. 30ml of dried pyridine was added to the oily product and cooled down with ice. 3.00g of trans-4-guanidinomethyl cyclohexane carboxylic acid · hydrochloride was added to the cooled mixture in portions. After stirring this mixture during night at room temperature, the mixture was concentrated under reduced pressure and acetone was added to the residue. Precipitated crystals were collected by filtration, and washed with acetone. 4.52g of trans-4-guanidinomethyl cyclohexane carboxylic acid (4-cyclohexylphenylester) · hydrochloride was obtained.
mp 205-206°C; NMR (DMSO-d₆) δ 0.8-3.3 (23H,m), 6.91 (2H,d), 7.17 (2H,d), 7.23 (4H,bs), 7.91 (1H,bt)

### Example 5

Cooling down with ice 1.67g of thionyl chloride was added by dropping to a mixture of 5.16g of 4-benzylphenol, 2.21g of pyridine and 30ml of dried tetrahydrofuran. After the mixture was stirred for 4 hours at room temperature, precipitated crystals were collected by filtration. The filtrate was concentrated under reduced pressure and an oily product was obtained. 30ml of dried pyridine was added to the oily product and cooled down with ice. 3.00g of trans-4-guanidinomethyl cyclohexane carboxylic acid · hydrochloride was added to the cooled mixture in portions. After stirring this mixture during night at room temperature, the mixture was concentrated under reduced pressure and acetone was added to the residue. Precipitated crystals were collected by filtration, and washed with acetone. 4.74g of crude crystals were obtained and the crystals were recrystallized with 28ml of 2-propanol 4.23g of trans-4-guanidinomethyl cyclohexane carboxylic acid (4-benzylphenylester) · hydrochloride was obtained.
mp 142-143°C; NMR (DMSO-d₆) δ 0.8-3.3 (12H,m), 3.90 (2H,s), 6.8-7.5 (13H,m), 7.93 (1H,bt)

### Example 6

Cooling down with ice, 1.67g of thionyl chloride was added by dropping to a mixture of 5.94g of 4-(1-methyl-1-phenylethyl)phenol, 2.21g of pyridine and 30ml of dried tetrahydrofuran. After the mixture was stirred for 4 hours at room temperature, precipitated crystals were collected by filtration. The filtrate was concentrated under reduced pressure and an oily product was obtained. 30ml of dried pyridine was added to the oily product and cooled down with ice. 3.00g of trans-4-guanidinomethyl cyclohexane carboxylic acid · hydrochloride was added to the cooled mixture in portions. After stirring this mixture during night at room temperature, the mixture was concentrated under reduced pressure and acetone was added to the residue. Precipitated crystals were collected by filtration, and washed with acetone. 4.92g of trans-4-guanidinomethyl cyclohexane carboxylic acid [4-(1-methyl-1-phenylethyl)phenylester] · hydrochloride was obtained.
mp 150-151°C; NMR (DMSO-d₆) δ 0.7-3.3 (12H,m), 1.63 (6H,s), 6.89 (2H,d), 7.0-7.4 (11H,m), 7.87 (1H,bt)

### Example 7

Cooling down with ice, 1.18g of thionyl chloride was added by dropping to a mixture of 5.13g of 4-(4-hydroxyphenyloxy)benzoate ethylester, 1.57g of pyridine and 30ml of dried tetrahydrofuran. After the mixture was stirred for 4 hours at room temperature, precipitated crystals were collected by filtration. The filtrate was concentrated under reduced pressure and an oily product was obtained. 30ml of dried pyridine was added to the oily product and cooled down with ice. 1.95g of trans-4-guanidinomethyl cyclohexane carboxylic acid · hydrochloride was added to the cooled mixture in portions. After stirring this mixture during night at room temperature, the mixture was concentrated under reduced pressure and acetone was added to the residue. Precipitated crystals were collected by filtration, and washed with acetone and hexane in this order. 2.95g of trans-4-guanidinomethyl cyclohexane carboxylic acid [4-(4-ethoxycarbonyl phenyloxy)phenylester] · hydrochloride was obtained.
mp 137-140°C; NMR (DMSO-d₆) δ 1.33 (3H,t), 0.8-3.3 (12H,m), 4.31 (2H,q), 7.04 (2H,d), 7.14 (4H,s), 7.28 (4H,bs), 7.98 (2H,d), 8.00 (1H,bs)

### Example 8

Cooling down with ice, 1.67g of thionyl chloride was added by dropping to a mixture of 5,61g of 4-benzyloxyphenol, 2,21g of pyridine and 30ml of dried tetrahydrofuran. After the mixture was stirred for 4 hours at room temperature, precipitated crystals were collected by filtration. The filtrate was concentrated under reduced pressure and crystals were obtained. 30ml of dried pyridine was added to the crystals and the mixture was cooled down with ice. 3.00g oftrans-4-guanidinomethyl cyclohexane carboxylic acid hydrochloride was added to the cooled mixture in portions. After stirring this mixture during night at room temperature, the mixture was concentrated under reduced pressure and acetone added to the residue. Precipitated crystals were collected by filtration, and washed with acetone. 4.76g of trans-4-guanidinomethyl cyclohexane carboxylic acid (4-benzyloxyphenylester) · hydrochloride was obtained.
mp 211-212°C; NMR (DMSO-d₆) δ 0.8-3.3 (12H,m), 5.07 (2H,s), 6.99 (4H,s), 7.27 (4H,bs), 7.40 (5H,s), 7.94 (1H,bt)

### Example 9

Cooling down with ice, 1.67g of thionyl chloride was added by dropping to a mixture of 5.50g of 4-styrylphenol, 2.21g of pyridine and 30ml of dried tetrahydrofuran. After the mixture was stirred for 6 hours at room temperature, precipitated crystals were collected by filtration. The filtrate was concentrated under reduced pressure and crystals were obtained. 80ml of dried pyridine was added to the crystals and the mixture was cooled down with ice. 3.00g of trans-4-guanidinomethyl cyclohexane carboxylic acid · hydrochloride was added to the cooled mixture in portions. After stirring this mixture during night at room temperature, the mixture was concentrated under reduced pressure and acetone was added to the residue. Precipitated crystals were collected by filtration, and washed with acetone. 4.89g oftrans-4-guanidinomethyl cyclohexane carboxylic acid (4-styrylphenylester) · hydrochloride was obtained.
mp 213-216°C; NMR (DMSO-d₆) δ 0.7-3.3 (12H,m), 6.7-8.2 (16H,m)

### Example 10

### Granules:

100 mg of trans-4-guanidinomethyl cyclohexane carboxylic acid (6-bromo-2-naphthylester) hydrochloride, that is the compound of Example 3, 80ml of milk sugar, 25ml of corn starch and 30ml of crystal cellulose were mixed well. Solution including hydroxy propylcellulose 15ml was added to the mixture. The mixture was kneaded, granulated, dried and classificated and granules were prepared.

**Table 1:**

| Compounds | MIC (µg/ml) | | |
|---|---|---|---|
| | Objected Strain 1 | Objected Strain 2 | Objected Strain 3 |
| Compound 1 | 3.13 | 0.78 | >25 |
| Compound 2 | 25 | 25 | >25 |
| Ophroxacine | 0.78 | 0.78 | 0.10 |
| Amoxicillin | 0.05 | 0.025 | 6.25 |

| | | | |
|---|---|---|---|
| Objected Strain 1: Helicobacter pylori ATCC 43504 Objected Strain 2: Helicobacter pylori ATCC 43629 Objected Strain 3: Escherichia coli NIH JC-2 | | | |

**Table 2**

| Compounds | MIC(µg/ml) | | |
|---|---|---|---|
| | Objected Strain 1 | Objected Strain 2 | Objected Strain 3 |
| Example 1 | 6.25 | 12.5 | >25 |
| Example 2 | 3.13 | 3.13 | >25 |
| Example 3 | 3.13 | 0.78 | >25 |
| Compound 3 | >25 | >25 | >25 |
| Compound 4 | 25 | 25 | >25 |

| | | | |
|---|---|---|---|
| Objected Strain 1: Helicobacter pylori ATCC 43504 Objected Strain 2: Helicobacter pylori ATCC 43629 Objected Strain 3: Escherichia coli NIH JC-2 | | | |

**Table 3**

| Compounds | MIC (µg/ml) | | |
|---|---|---|---|
| | Objected Strain 1 | Objected Strain 2 | Objected 3 |
| Example 4 | 3.13 | 0.78 | >25 |
| Example 5 | 12.5 | 12.5 | >25 |
| Example 6 | 25 | 25 | >25 |
| Example 7 | 6.25 | 6.25 | >25 |
| Example 8 | 12.5 | 3.13 | >25 |
| Example 9 | 0.78 | 0.39 | >25 |
| Ophroxacine | 0.78 | 0.78 | 0.10 |
| Amoxicillin | 0.05 | 0.025 | 6.25 |

| | | | |
|---|---|---|---|
| Objected Strain 1: Helicobacter pylori ATCC 43 504 Objected Strain 2: Helicobacter pylori ATCC 43629 Objected Strain 3: Escherichia coli NIH JC-2 | | | |

**Table 4**

| Compound | MIC (µg/ml) | | | |
|---|---|---|---|---|
| | Strain 11 | Strain 12 | Strain 13 | Strain 14 |
| Example 7 | 6.25 | | 25 | 6.25 |
| Ophroxacine | 0.39 | 0.10 | 0.10 | 12.5 |
| Amoxicillin | 0.10 | 0.05 | 0.05 | 0.20 |

| | | | | |
|---|---|---|---|---|
| * Strain means Object strain. * Strain 11: Staphylococcus aureus 209PJC Strain 12: Staphylococcus aureus ATCC 6538 Strain 13: Bacillus subtilis ATCC 6633 Strain 14: Staphylococcus aureus No. 2 (MRSA) | | | | |

## Claims

1. A compound shown in the Formula (III) 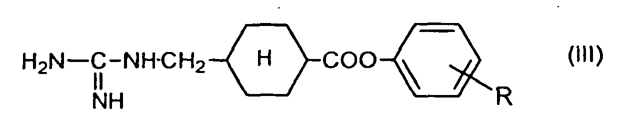 (In the formula, R is selected from the group consisting of cycloalkyl group having three to eighteen carbon atoms, aralkyl group having seven to eighteen carbon atoms, arylalkenyl group having eight to eighteen carbon atoms, and substituted phenoxy group) or pharmaceutically acceptable salts thereof.

2. A compound shown in the Formula (IV) 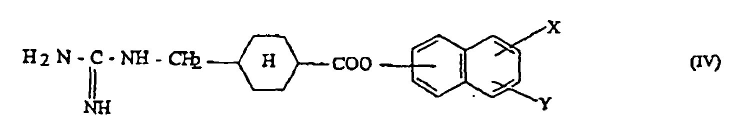 (In the formula, X and Y axe selected from the group consisting of hydrogen, and alkoxycarbonyl group having two to nineteen carbon atoms respectively, with the proviso that X and Y are not hydrogen) or pharmaceutically acceptable salts thereof.

3. A compound selected from the group consisting of trans-4-guanidinomethyl cyclohexane carboxylic acid (6-ethoxycarbonyl-2-naphthylester), trans-4-guanidinomethyl cyclohexane carboxylic acid (2,4-dichloro-1-naphthylester), trans-4-guanidinomethyl cyclohexane carboxylic acid (6-bromo-2-naphthylester), trans-4-guanidinomethyl cyclohexane carboxylic acid (4-cyclohexylphenylester), and trans-4-guanidinomethyl cyclohexane carboxylic acid (4-styrylphenylester), and pharmaceutically acceptable salts thereof.

## Patentansprüche

1. Verbindung der Formel (III) 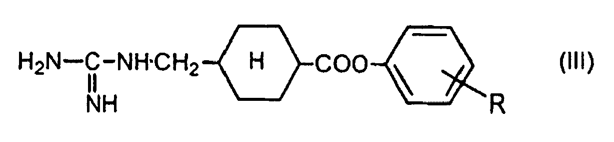 (In der Formel wird R ausgewählt aus der Gruppe, bestehend aus einer Cycloalkylgruppe mit drei bis achtzehn Kohlenstoffatomen, einer Aralkylgruppe mit sieben bis achtzehn Kohlenstoffatomen, einer Arylalkenylgruppe mit acht bis achtzehn Kohlenstoffatomen und einer substituierten Phenoxygruppe) oder pharmazeutisch verträgliche Salze davon.

2. Verbindung der Formel (IV) 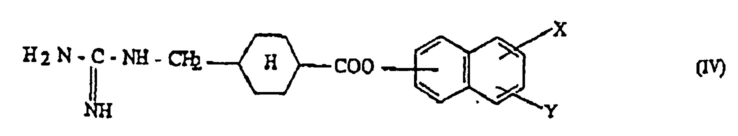 (In der Formel werden X und Y ausgewählt aus der Gruppe, bestehend aus einem Wasserstoffatom und einer Alkoxycarbonylgruppe mit zwei bis neunzehn Kohlenstoffatomen, mit der Maßgabe, dass X und Y nicht beide Wasserstoffatome sind) oder pharmazeutisch verträgliche Salze davon.

3. Verbindung, ausgewählt aus der Gruppe, bestehend aus trans-4-Guanidinomethylcyclohexancarbonsäure(6-ethoxycarbonyl-2-naphthylester), trans-4-Guanidinomethylcyclohexancarbonsäure (2,4-dichlor-1-naphthylester), trans-4-Guanidinomethylcyclohexancarbonsäure(6-brom-2-naphthylester), trans-4-Guanidinomethylcyclohexancarbonsäure(4-cyclohexylphenylester) und trans-4-Guanidinomethylcyclohexancarbonsäure(4-styrylphenylester), und pharmazeutisch verträgliche Salze davon.

## Revendications

1. Composé représenté par la formule (III) 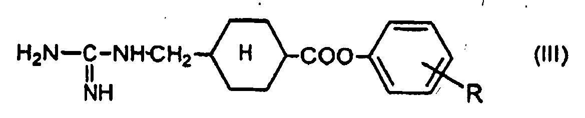 (dans la formule, R est choisi dans le groupe consistant en un groupe cycloalkyle ayant de trois à dix-huit atomes de carbone, un groupe aralkyle ayant de sept à dix-huit atomes de carbone, un groupe arylalcényle ayant de huit à dix-huit atomes de carbone, et un groupe phénoxy substitué) ou ses sels pharmaceutiquement acceptables.

2. Composé représenté par la formule (IV) 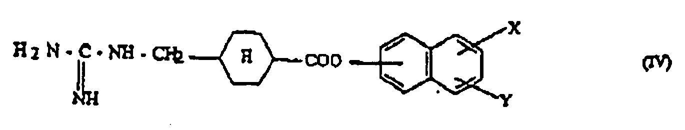 (dans la formule, X et Y sont choisis dans le groupe consistant en l'hydrogène, et un groupe alcoxycarbonyle ayant de deux à dix-neuf atomes de carbone respectivement, avec pour condition que X et Y ne sont pas tous deux l'hydrogène) ou ses sels pharmaceutiquement acceptables.

3. Composé choisi dans le groupe consistant en ester 6-éthoxycarbonyl-2-naphtylique d'acide trans-4-guanidinométhylcyclohexanecarboxylique, ester 2,4-dichloro-1-naphtylique d'acide trans-4-guanidinométhylcyclohexanecarboxylique, ester 6-bromo-2-naphtylique d'acide trans-4-guanidinométhylcyclohexanecarboxylique, ester 4-cyclohexylphénylique d'acide trans-4-guanidinométhylcyclohexanecarboxylique, et ester 4-styrylphénylique d'acide trans-4-guanidinométhylcyclohexanecarboxylique, et ses sels pharmaceutiquement acceptables.
